Europäisches Patentamt

**European Patent Office**

**Office européen des brevets**

(11) Publication number: **0 301 122 B1**

# EUROPEAN PATENT SPECIFICATION

(45) Date of publication of patent specification: **28.10.92**

(51) Int. Cl.5: **A61K 37/64**, A61K 47/00, A61K 45/06

(21) Application number: **87111047.4**

(22) Date of filing: **30.07.87**

The file contains technical information submitted after the application was filed and not included in this specification

(54) **Use of soybean kunitz type trypsin and derivatives in the preparation of medicaments and agent comprising the same.**

(43) Date of publication of application:
**01.02.89 Bulletin 89/05**

(45) Publication of the grant of the patent:
**28.10.92 Bulletin 92/44**

(84) Designated Contracting States:
**DE FR GB**

(56) References cited:
**US-A- 3 853 708**

**CHEMICAL ABSTRACTS, vol. 103, no. 3, 22nd July 1985, page 476, abstract no. 21359j, Columbus, Ohio, US; J. YAVELOW et al.: "In vitro effects of soybean protease inhibitors"**

**CHEMICAL ABSTRACTS, vol. 88, no. 23, 5th June 1978, page 39, abstract no. 163955w, Columbus, Ohio, US; R. VERLOES et al.: "Tumor growth inhibition mediated by trypsin inhibitor or urokinase inhibitors", & EUR. J. CANCER 1978, 14(1), 23-31**

(73) Proprietor: **Maeda, Hiroshi**
**631-3, Hotakubo-honmachi**
**Kumamoto-shi Kumamoto-ken(JP)**

Proprietor: **FUJI OIL COMPANY, LIMITED**
**No. 6-1, Hachiman-cho Minami-ku**
**Osaka-shi Osaka(JP)**

(72) Inventor: **Maeda, Hiroshi**
**No. 631-3, Hotakubo-honmachi**
**Kumamoto-shi Kumamoto(JP)**
Inventor: **Matsumura, Yasuhiro**
**No. 212, Yoyasub-cho**
**Kumamoto-shi Kumamoto(JP)**
Inventor: **Takamatsu, Kiyoharu**
**No. 1-13-10, Tsuru-machi Taisyou-ku**
**Osaka-shi Osaka(JP)**
Inventor: **Shimoda, Tadahisa**
**No. 411-244, Shichiyama Kumatori-cho**
**Sennan-gun Osaka(JP)**

CHEMICAL ABSTRACTS, vol. 86, no. 19, 9th May 1977, page 48, abstract no. 133639n, Columbus, Ohio, US; R. VERLOES et al.: "Proteolytic activity associated with tumor growth and metastasis. Influence of trypsin inhibitor (soybean) on Ehrlich ascites tumor growth", & ARCH. INT. PHYSIOL. BIOCHIM. 1976, 84(5), 1119-20

BIOLOGICAL ABSTRACTS, vol. 78, no. 9, 1984, abstract no. 69854, Biological Abstracts Inc., Philadelphia, US; H. BEKEMEIER et al.: "Mediators and inhibitors of foot edema induced by carrageenan, dextran and trypsin, and adjuvant arthritis in the rat", & WISS Z MARTIN LUTHER UNIV HALLE-WITTENBERG MATH-NATURWISS REIHE 33(2), 3-22, 1984

BIOLOGICAL ABSTRACTS, vol. 63, 15th May 1977, abstract no. 60288, Biological Abstracts, Inc., Philadelphia, US; A.M. ROTH-SCHILD et al.: "Contribution of vasopressor and plasma kininogen changes towards acute adrenaline pulmonary edema in the rat", & NAUNYN-SCHMIEDEBERG'S ARCH PHARMACOL 295(2), 177-181, 1976

JOURNAL OF CELLULAR BIOCHEMISTRY SUPPLEMENT, vol. 0, no. 9, part A, 1985, page 56; J.-Y. LIN et al.: "New antitumor chimeric protein: CON A-soybeam trypsin inhibitor conjugate"

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

## Description

This invention relates to an agent for inhibiting retention of cancerous hydrothorax and ascites, an agent for inhibiting inflammatory edema exaggeration, and an agent for enhancing carcinostatic activities.

Various protease inhibitors of plant origin have hitherto been reported. These inhibitors have specificity. For example, it is known that a corn trypsin inhibitor and a pumpkin trypsin inhibitor exert their inhibitory activity upon trypsin and an active Hageman factor as described in Thromb. Res., Vol. 20, 149 (1980) and that Kunitz type trypsin inhibitor of soybean trypsin inhibitors has an inhibitory effect on trypsin, plasma kallikrein and activated Factor X.

Exaggeration of inflammatory edema is associated with manifestation of bradykinin activity to increase pain or permeability of vessels. A scheme of the manifestation of bradykinin has been proposed, in which a Hageman factor or kallikrein takes part.

However, studies on the relationship between these protease inhibitors and inflammation are so far confined to in vitro experimentations, and there have been reported only a few cases illustrative of the effects in vivo.

On the other hand cancer-bearing patients often suffer from hydropsy, i.e., retention of ascites in case of gastric cancer, renal cancer, ovarian cancer and hepatic cancer, or retention of hydrothorax in case of lung cancer. Such ascites or hydrothorax disseminates solid tumors, after the operation, to cause metastatic tumors in the abdominal cavity or the thoracic cavity at a high probability, which results in similar ascitic or hydrothoracic retention. Ascitic retention brings about reduction of plasmaprotein, leading to declination of physical strength of the patients or other aggravations. As a result, the therapeutic effects are lessened, and transfusion of a large quantity of blood would be required. Hydrothoracic retention causes respiration insufficiency and ultimately brings very grave results, such as accelerated cachexia.

The inventors have conducted extensive investigations on the relationship between protease inhibitors and inflammation inhibition. As a result, it has now been found that soybean Kunitz type trypsin inhibitor and, inter alia, its derivatives produce marked effects on inhibition of inflammatory edema in vivo. The inventors have further investigated, with their attention being focused on the similarity in mechanism between increase of vascular permeability due to inflammation and that due to solid tumors, and more particularly similarity between inflammatory sites and solid tumors in terms of exaggeration of vascular permeability and extravasation of plasma components. As a result, it has also been found that the aforesaid inhibitor and derivatives thereof are effective to suppress retention of cancerous ascites or hydrothorax. That is, it has been experimentally confirmed that continuous administration of the aforesaid inhibitors to an ascites-tumor system (Meth A. and Sarcoma 180) remarkably inhibits retention of ascites due to their strong inhibitory activity on vascular permeability in cancer tissues to thereby prolong the survival time of cancer-bearing mice. Therefore, these inhibitors would be promising in the treatment of cancers. The present invention has been completed based on these findings.

The present invention relates to an agent for inhibiting retention of cancerous ascites or hydrothorax and an agent for inhibiting exaggeration of inflammatory edema, comprising soybean Kunitz type trypsin inhibitor or a derivative thereof as an active ingredient, wherein said derivative is obtained by modifying soybean Kunitz type trypsin inhibitor with a styrene-maleic anhydride copolymer or a partial ester thereof, polyethylene glycol, a vinyl ether copolymer, a pyran or a dextran.

The present invention also relates to an agent for enhancing activities of carcinostatic substance, which comprises soybean Kunitz type trypsin inhibitor or a derivative thereof as an active ingredient, wherein said derivative is obtained as stated above, and wherein said carcinostatic substance is selected from the group of anticancerous antibiotics, metabolic antagonists, immune activators and anticancer agents.

The invention relates also to the use of the above mentioned active ingredients in the preparation of a medicament.

Figures 1 and 2 each is a graph showing the inhibitory effects of drugs of Example 2 on body weight gain due to ascitic retention.

Figure 3 is a graph showing the change of volume of the edema according to Example 4.

Soybean trypsin inhibitors are roughly divided into Kunitz type trypsin inhibitors having a molecular weight of about 20,000 and Bowman-Birk type trypsin inhibitors having a molecular weight of from 6,000 to 8,000. Basic properties of these inhibitors have been elucidated as described, e.g., in Methods in Enzymology, Vol. 19, 853 (1970) and Tanpakushitsu Kenkyu no Atarashii Shiten "Kagakuteki Kenkyu o Chushin to shite" (New Viewpoint of Studies on Proteins "centered at chemical studies"), 1738, Kyoritsu Shuppan (1982). Chemical structures of typical examples of the Kunitz type trypsin inhibitors (hereinafter referred to as KTI) and Bowman-Birk type trypsin inhibitors (hereinafter referred to as BBI) have already been established so that these inhibitors can be synthesized nowadays through chemical processes or biological processes, such as tissue culture and gene recombination.

The agents according to the present invention comprise KTI or a derivative thereof as an active ingredient, wherein said derivative is obtained by modifying soybean Kunitz type trypsin inhibitor with a styrene-maleic anhydride copolymer or a partial ester thereof, polyethylene glycol, a vinyl ether copolymer, a pyran or a dextran. KTI to be used embraces KTI as isolated from soybeans and products of limited degradation of isolated soybean KTI while retaining activities inherent to KTI, as well as KTI synthesized by the above-mentioned chemical or biological processes.

KTI of soybean origin can be prepared by any of the known processes. According to a commonly employed process, raw materials, such as soybeans, defatted soybeans and soybean whey are extracted with aqueous media or polar organic solvents (e.g., ethanol and acetone), and the extract is subjected to concentration and fractionation techniques, such as membrane separation, I.E.P. precipitation, and salting out, to obtain a crude product, which is then purified by gel filtration, ion exchange, physical or chemical adsorption. The activities and purities of KTI can be determined by known methods, for example, a casein digestion method by Kunitz and electrophoresis analysis using a SDS-containing polyacrylamide gel. In the present invention, the former method is adopted, and the amount that inhibits 1 mg of Type-XI (trypsin produced by Sigma Co.; active protein having 7500 to 9000 BAEE unit/mg) is taken as 1 unit.

The effective amount of the active ingredient somewhat varies depending on the degree of purification, the administration route, and the residual activity, and usually ranges from 1 to 3000 mg/60 Kg-body weight in intraperitoneal administration or from 1 to 300 mg/60 Kg-body weight in intravenous injection as converted to KTI protein weight having an activity of from 1.5 to 2.5 unit/mg.

The derivatives of KTI can be used in the present invention for one or more purposes, such as reduction of antigenicity of KTI protein, enhancement of stability to decomposing substances (e.g., SH-protease), i.e., improvement of in vivo half-life period and impartment of hydrophobic properties to increase amphiphatic properties. Such KTI derivatives are combinations of KTI and various modifiers, i.e., styrene-maleic anhydride copolymers (SMA) and partial esters thereof, polyethylene glycol, vinyl ether copolymers, pyrans and dextrans. The manner for combining KTI with these modifiers may be any of conventionally known methods as long as the purpose or purposes intended can be accomplished. For example, in case of modifying with SMA or partial esters thereof, the modifier is directly bound to an $\epsilon$-amino group of from the N-terminated amino group to an $\epsilon$-amino group of the lysine residue of KTI in a neutral to alkaline solution to form a peptide linkage as reported by Maeda, et al., the inventors of the present invention, in Journal of Medicinal Chemistry, Vol. 28, 455-461 (1985).

KTI and derivatives thereof can be administrated topically, intravenously, intraarterially, permucously, percutaneously, orally or intraperitoneally.

For example, water-soluble preparations for intravenous injection include KTI as isolated and injectable solutions of KTI in physiological saline or a 5 wt% glucose solution. Preparations for intraarterial injection include oils using lipiodol, emulsions prepared by emulsifying an aqueous solution of KTI in fats and oils, e.g., olive oil, medium chain fatty acid esters, linolic esters, soybean oil and tung oil, in the presence of an emulsifier, and forms of liposome.

KTI or derivatives thereof can be used in combination with drugs having carcinostatic activity per se. Examples of the carcinostatics to be used in combination include anticancerous antibiotics, e.g., Neocarzinostatin (NCS), SMANCS (a styrene-maleic acid copolymer bound to NCS via the amino group of NCS) and Mitomycin (trade mark), metabolic antagonists, e.g., 5-FU (trade mark), immune activators, e.g., Picibanil (trade mark), and other anticancer agents. These carcinostatics may be combined with KTI or its derivatives in a single dose form, or the carcinostatics and KTI or its derivatives may be administered separately.

KTI and its derivatives according to the present invention exhibit effects to inhibit exaggeration of inflammatory edema and retention of ascites and hydrothorax to the ultimate advantage of prolonging the survival time. KTI and its derivatives are also effective to enhance anticancer activities possessed by various carcinostatics when used in combination therewith. The mechanism of production of these effects is not completely clarified, but it is assumed that KTI effectively inhibits a kallikrein-bradykinin system in vivo that is believed to participate in inflammatory edema to thereby suppress pain generation, and acceleration of vascular permeability due to manifestation of bradykinin; that KTI inhibits permeability of vessels in cancer tissues, in which the above-described system is present, i.e., extravasation of plasmaproteins, into cancer tissues is inhibited, to thereby inhibit retention of ascites or hydrothorax; and that KTI shuts off the supply of plasma components (nutrition) to the cancer tissues, which leads to enhancement of the activities possessed by anticancer agents. There is a possibility that KTI partly inhibits the activity of proteases which accelerate growth of cancer cells. Since KTI has a molecular weight of about 20,000 (KTI derivatives have higher molecular weights), it selectively acts on those tissues having exaggerated vascular permeability to inhibit growth of the edema or cancer tissue without substantially undergoing extravasation into normal tissues where vascular permeability is not exaggerated. Further, while the exudate (extravasation) at the inflammatory site is usually recovered through lymphatic vessels, since there is no lymphatic system in cancer tissues, the high polymeric substances extravasated in the cancer tissues are considered to be retained there for a prolonged period of time without being recovered. It is believed that this property also contributes to the selective action of KTI on the affected site.

The above-described shut-off of a nutrition supply to cancer tissues is similar to the concept of inhibition of a nutrition supply by physical embolic therapy. However, the physical embolic therapy often involves destruction of normal tissues. On the other hand, the agent according to the present invention does not cause such adverse effects.

The present invention will now be illustrated in greater detail by way of the following examples.

EXAMPLE 1

Preparation of KTI:

Soybean whey obtained in the process for isolating soybean protein from low-denatured defatted soybeans was concentrated so as to have a crude protein content of 5.5 wt%. Half as much acetone as the concentrate by volume was added thereto, and the mixture was stirred for about 1 hour, followed by centrifugal separation. To the supernatant liquor was further added one and half the volume of acetone as much as the above concentrate, followed by stirring for about an additional one hour. The precipitated fraction separated by centrifugation was subjected to dialysis against water. To the dialysate was added a 0.5M sodium phosphate buffer solution (pH 7.0) in an amount of 1/50 by volume to adjust to a pH of 7.0, and the system was passed through an ion-exchange column packed with DEAE cellulose to adsorb the active ingredient onto the resin. The column was then eluted with an eluent containing sodium chloride having a straight concentration gradient of from 0 M up to 0.4 M and the eluent was collected by means of an automatic fraction collector. Each of the combined active fraction rich in BBI and the combined active fraction rich in KTI was salted out and concentrated. The concentrate of BBI was further purified by a CM cellulose ion-exchange resin and lyophilized. The KTI fraction was subjected to I.E.P. precipitation and lyophilization to obtain a KTI sample. Both the samples had a purity of 95% or more in the total protein as determined by SDS-containing polyacrylamide gel electrophoresis. The KTI sample and the BBI sample had a specific activity of 1.97 unit/mg-protein and 3.37 unit/mg-protein, respectively.

EXAMPLE 2

Inhibition of Ascitic Retention and Prolongation of Life:

A suspension containing 500,000 cells/animal of Sarcoma 180 was intraperitonearlly inoculated to seven 8-week-old female ddY mice per group. A water-soluble preparation prepared by dissolving 3 mg of the KTI or BBI as obtained in Example 1 in 1 ml of physiological saline or physiological saline containing no trypsin inhibitor (blank) was intraperitonearlly administered to each of the test animals at a level of 1 ml/day for consecutive 4 days or 14 days from the day of inoculation. The animals having received no inoculation of Sarcoma 180 and drugs were used as the control group. During the administration period, the animals were allowed food and water ad libitum.

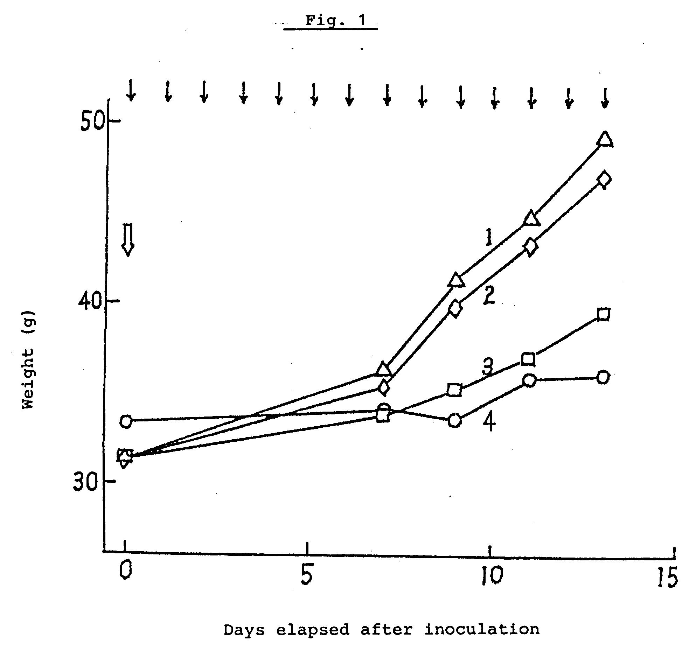

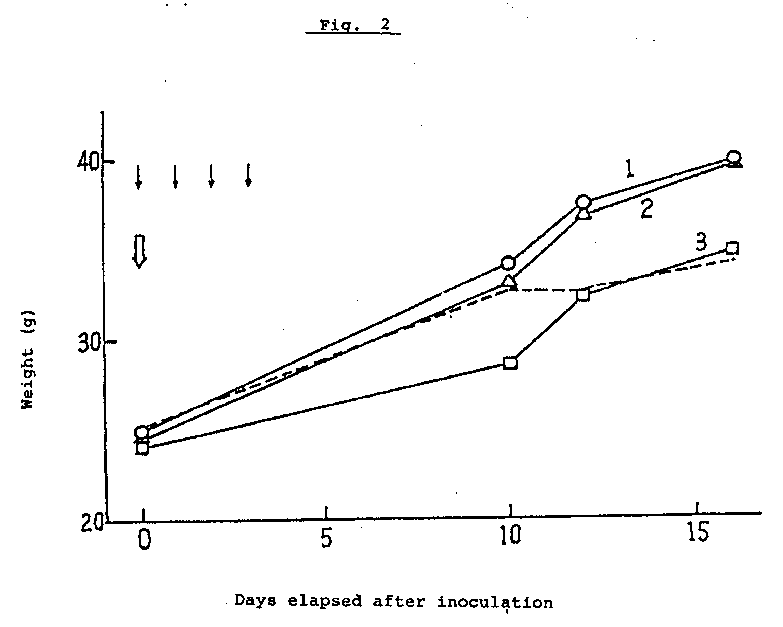

The animals were weighed every day during the administration, and the results of weight gain are plotted in Figure 1 (14 days' administration) and Figure 2 (4 days' administration). In Figs. 1 and 2, plots 1 indicate the group receiving physiological saline; plots 2 indicate the group receiving BBI; plots 3 indicate the group receiving KTI; plots 4 indicate the control group; white arrow indicates the day of inoculation of cancer cells; and the black arrows indicate the days of administration of the drug. In Fig. 2, the dotted line indicates the control group. In view of the results shown in Figs. 1 and 2 combined with visual observation, the group receiving KTI obviously demonstrates the effect of weight gain inhibition, i.e., inhibition of ascitic retention, showing no substantial difference from the control group, whereas such an inhibitory effect is insubstantial in the group receiving BBI.

An average of days of survival and a 20-day survival rate is shown in Table 1 below. It can be seen from the Table that KTI is effective to prolong the life.

## TABLE 1

| Group | 14 Days' Admn. | | 4 Days' Admn. |
| | Average days of Survival | 20-Days Survival Rate | Average days of Survival |
|---|---|---|---|
| Blank Group | 19.1 days | 28.6% | 21.4 days |
| KTI Group | 26.0 days | 100.0% | 24.8 days |
| BBI Group | 21.9 days | 71.4% | 21.0 days |

EXAMPLE 3

Preparation of KTI Bound to Partially Butylated SMA:

In 30 ml of a 0.5M sodium hydrogencarbonate aqueous solution was dissolved 300 mg of KTI as prepared in Example 1, and 150 mg of partially butylated SMA (average molecular weight: ca. 2,000), and the solution was stirred at 25°C for 90 minutes. A glycine solution was added to the reaction system to stop the reaction. The resulting solution had a residual activity of 36.3% and contained 1.94 modified amino groups per molecule as determined by a TNBS method [cf. Tanpakushitsu, Kakusan, Koso (Proteins, Nucleic Acids, and Enzymes), Vol. 18 (13), 1153-1159 (1973)]. The solution was dialyzed against an ammonium hydrogencarbonate aqueous solution, followed by lyophilization to obtain an SMA-modified KTI (hereinafter referred to as KTI-SMA).

EXAMPLE 4

Inhibition of Inflammatory Edema Exaggeration:

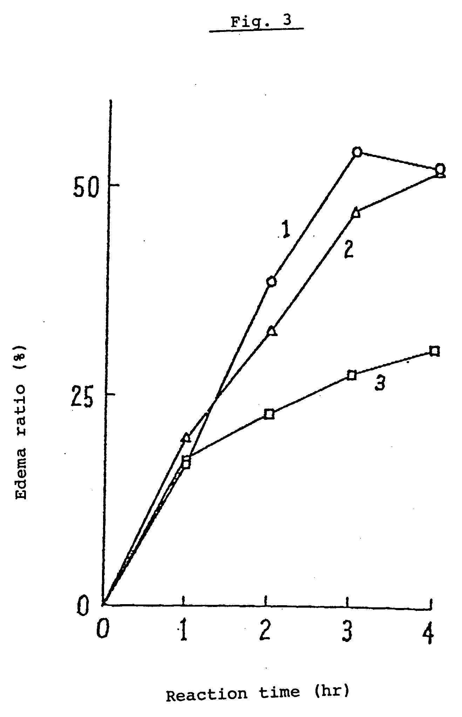

A 1 wt% carrageenin solution was prepared by dissolving carrageenin in physiological saline (blank) or physiological saline containing 5 mg or 15 mg/ml of KTI-SMA as prepared in Example 3. Five male SD rats (average body weight: 150 g ± 5 g) per group were locally administered with 0.1 ml/site (paw) of the carrageenin solution to induce an inflammation at the paw. The volume of the paw was measured with time to determine the inhibitory effect on carrageenin-induced edema. The results obtained are plotted in Figure 3. In Fig. 3, plots 1 indicate the blank group; plots 2 indicate the group having received 5 mg/ml of KTI-SMA; and plots 3 indicate the group having received 15 mg/ml of KTI-SMA.

As can be seen from Fig. 3, a significant effect of inflammatory edema inhibition is noted in the group having received 15 mg/ml of KTI-SMA after the elapse of 3 hours from the inflammation induction.

When the same test was repeated, except for replacing KTI-SMA with 20 mg/ml of unmodified KTI, it was revealed that the inhibitory effect was almost equal to that produced by 5 mg/cc of KTI-SMA.

EXAMPLE 5

A Sarcoma 180 suspension (500,000 cells/animal) was intraperitoneally implanted to ten 8-week-old female ddY mice per group. The test animals were administered with physiological saline containing KTI as prepared in Example 1, neocarzinostatin (NCS) or a mixture of KTI and NCS once a day for consecutive 7 days. A control group received physiological saline only. During the experimentation, the animals were allowed food and water ad libitum. The doses and changes of the survival rate are shown in Table 2 below.

6

## TABLE 2

| Group | Dose (mg/Kg-b.d.) | Survival Rate | | |
|---|---|---|---|---|
| | | 20-Days (%) | 30-Days (%) | 50-Days (%) |
| Control | – | 10 | 0 | 0 |
| KTI | 100 (a) | 100 | 30 | 0 |
| NCS | 0.01 (b) | 100 | 30 | 0 |
| KTI + NCS | (a) + (b) | 100 | 60 | 40 |

As is apparent from Table 2, KTI not only exhibits per se an activity to prolong the life but also exerts synergistic effects with a carcinostatic (NCS).

As described above, agents comprising KTI or a derivative thereof as an active ingredient exert effects to inhibit inflammatory edema, effects to inhibit retention of ascites or hydrothorax, as well as effects to enhance carcinostatic activity. Inhibition of inflammatory edema prevents generation or exaggeration of pain due to edema, and inhibition of ascitic or hydrothoracic retention prevents or suppresses malignant influences associated therewith, such as reduction of physical strength of patients, reduction of therapeutic effects, necessity of transfusion of a large quantity of blood plasma, respiration insufficiency and introduction of cachexia, and enhances the therapeutic effects, thus resulting in prolongation of the life.

## Claims

1. Soybean Kunitz type trypsin inhibitor or a derivative thereof as an active ingredient, wherein said derivative is obtained by modifying soybean Kunitz type trypsin inhibitor with a styrene-maleic anhydride copolymer or a partial ester thereof, polyethylene glycol, a vinyl ether copolymer, a pyran or a dextran, for use as an agent for inhibiting retention of cancerous ascites and/or hydrothorax and inhibiting exaggeration of inflammatory edema.

2. Soybean Kunitz type trypsin inhibitor or a derivative thereof as an active ingredient, wherein said derivative is obtained by modifying soybean Kunitz type trypsin inhibitor with a styrene-maleic anhydride copolymer or a partial ester thereof, polyethylene glycol, a vinyl ether copolymer, a pyran or a dextran, for use as an agent for enhancing activities of carcinostatic substance, wherein said carcinostatic substance is selected from anticancerous antibiotics, metabolic antagonists, immune activators and anticancer agents.

3. Use of the active ingredients of claim 1 in the preparation of a medicament for inhibiting retention of cancerous hydrothorax and ascites.

4. Use of the active ingredients of claim 1 in the preparation of a medicament for inhibiting inflammatory edema exaggeration.

5. Use of the active ingredients of claim 1 in the preparation of a medicament for enhancing the activity of carcinostatic substances.

6. Use according to claim 5 in which the carcinostatic substance is selected from the group listed in claim 2.

## Patentansprüche

1. Sojabohnen-Kunitz-Typ-Trypsininhibitor oder ein Derivat davon als ein aktiver Bestandteil, wobei das Derivat erhalten ist durch Modifizieren eines Sojabohnen-Kunitz-Typ-Trypsininhibitors mit einem Styrol-Maleinsäureanhydrid-Copolymer oder einem teilweisen Ester davon, Polyethylenglycol, einem Vinylethercopolymer, einem Pyran oder einem Dextran, für die Verwendung als ein Mittel zur Inhibition der Retention von cancerogenem Ascites und/oder Hydrothorax und zur Inhibition der übermäßigen Bildung von entzündlichen Ödemen.

**2.** Sojabohnen-Kunitz-Typ-Trypsininhibitor oder ein Derivat davon als ein aktiver Bestandteil, wobei das Derivat erhalten ist durch Modifizieren von Sojabohnen-Kunitz-Typ-Trypsininhibitor mit einem Styrol-Maleinsäureanhydrid-Copolymer oder einem teilweisen Ester davon, Polyethylenglycol, einem Vinylethercopolymer, einem Pyran oder einem Dextran, für die Verwendung als ein Mittel zur Erhöhung der Aktivitäten einer carcinostatischen Substanz, wobei die carcinostatische Substanz ausgewählt ist aus anticancerogenen Antibiotika, metabolischen Antagonisten, Immunaktivatoren und Antikrebsmitteln.

**3.** Verwendung der aktiven Bestandteile nach Anspruch 1, für die Herstellung eines Medikamentes zur Inhibition der Retention von cancerogenem Hydrothorax und Ascites.

**4.** Verwendung der aktiven Bestandteile nach Anspruch 1, zur Herstellung eines Medikamentes zur Inhibition der übermäßigen Bildung von entzündlichen Ödemen.

**5.** Verwendung der aktiven Bestandteile nach Anspruch 1, für die Herstellung eines Medikamentes zur Vergrößerung der Aktivität von carcinostatischen Substanzen.

**6.** Verwendung nach Anspruch 5, wobei die carcinostatische Substanz aus der Gruppe ausgewählt wird, die in Anspruch 2 angegeben ist.

**Revendications**

**1.** Inhibiteur de la trypsine de type Kunitz du soja ou un dérivé de celui-ci en tant qu'ingrédient actif, où ledit dérivé est obtenu par modification d'un inhibiteur de la trypsine de type Kunitz du soja avec un copolymère de styrène-anhydride maléique ou un ester partiel de celui-ci, un polyéthylèneglycol, un copolymère d'éther vinylique, un pyranne ou un dextran, pour l'emploi comme agent pour inhiber l'ascite et/ou l'hydrothorax cancéreux et inhiber l'exagération de l'oedème inflammatoire.

**2.** Inhibiteur de la trypsine de type Kunitz du soja ou un dérivé de celui-ci en tant qu'ingrédient actif, où ledit dérivé est obtenu par modification d'un inhibiteur de la trypsine de type Kunitz du soja avec un copolymère de styrène-anhydride maléique ou un ester partiel de celui-ci, un polyéthylèneglycol, un copolymère d'éther vinylique, un pyranne ou un dextran, pour l'emploi comme agent pour accroître les activités d'une substance cancérostatique, où ladite substance cancérostatique est choisie parmi les antibiotiques anticancéreux, les antagonistes métaboliques, les activateurs de l'immunité et les agents anticancéreux.

**3.** Emploi des ingrédients actifs de la revendication 1 dans la préparation d'un médicament pour inhiber l'hydrothorax et l'ascite cancéreux.

**4.** Emploi des ingrédients actifs de la revendication 1 dans la préparation d'un médicament pour inhiber l'exagération de l'oedème inflammatoire.

**5.** Emploi des ingrédients actifs de la revendication 1 dans la préparation d'un médicament pour accroître l'activité des substances cancérostatiques.

**6.** Emploi selon la revendication 5, dans lequel la substance cancérostatique est choisie dans le groupe indiqué dans la revendication 2.

Fig. 1

Fig. 2

EP 0 301 122 B1

Fig. 3